# FASCICULE DE BREVET EUROPEEN

(11) **EP 0 344 044 B1**
(45) Date de publication et mention de la délivrance du brevet: **15.06.1994**
(21) Numéro de dépôt: 89401339.0
(22) Date de dépôt: 12.05.1989
(51) Int. Cl.: C12P 41/00, C12P 7/40, C12P 11/00

(54) **Procédé de préparation d'acides aryloxy-2 ou arylthio-2 alkanoiques optiquement actifs**
Verfahren zur Herstellung von optisch aktiven 2-Aryloxy- oder 2-Arylthioalkansäuren
Process for the preparation of optically active 2-aryloxy- or 2-arylthioalcanoic acids

(30) Priorité: 26.05.1988 FR 8807009
(43) Date de publication de la demande: 29.11.1989
(73) Titulaire: RHONE-POULENC AGROCHIMIE, 69263 Lyon Cedex 09 (FR)
(72) Inventeur: Petre, Dominique, F-69006 - Lyon (FR); Cerbelaud, Edith, F-69005 - Lyon (FR)
(74) Mandataire: Trolliet, Maurice

(56) Documents cités:
- EP-A- 0 214 569
- WO-A-86/07386
- FR-A- 1 342 844

## Description

La présente invention concerne un procédé de préparation d'acides aryloxy-2 ou arylthio-2 alkanoïques optiquement actifs de formule générale : 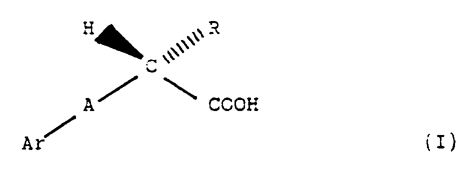
par hydrolyse énantiosélective des amides racémiques correspondants
Dans la formula (I)
. Ar représente un radical aromatique éventuellement substitué par au moins, un groupe alkyle, alkoxy, halogéno, hydroxyle, les groupes alkyles ou alkoxy ayant de préférence 1 à 4 atomes de carbone;
. A représente un atome d'oxygène ou de soufre;
. R représente un radical alkyle ayant 1 à 6 atomes de carbone.

Plus particulièrement, la présente invention concerne la préparation des énantiomères R (+) des acides aryloxy-2 ou arylthio-2 alkanoïques de formule générale (I).

Plus particulièrement encore, la présente invention concerne la préparation des énantiomères R (+) des acides phénoxy-2 ou phénylthio-2 propioniques de formule générale : 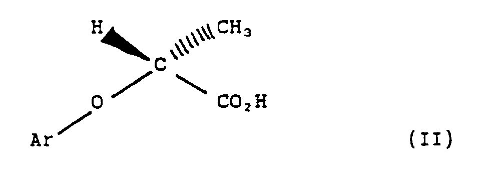
dans laquelle Ar a la même signification que dans la formule I.

La présente invention a encore plus particulièrement pour objet la préparation de l'acide (hydroxy-4, phénoxy)-2 propionique R(+).

Il a maintenant été trouvé, et c'est ce qui fait l'objet de la présente invention, que les acides aryloxy-2 ou arylthio-2 alkanoïques de formule générale (I) sous forme R peuvent être obtenus par hydrolyse énantiosélective des amides racémiques correspondants, éventuellement préparés in situ, au moyen d'un microorganisme ou d'une enzyme issue de ce microorganisme qui :
- est choisi parmi les souches appartenant aux genres Brevibacterium et Corynbacterium, et
- à la propriété de posséder une activité nitrilase associée à la capacité d'hydrolyser énantiosélectivement l'α -phényl propionamide racémique en acide -α phénylpropionique S(+) avec un excès énanthiomérique supérieur à 65 %.

La sélection des agents selon l'invention permettant l'hydrolyse énantiosélective des amides des acides aryloxy-2 ou arylthio-2 alkanoïques de formule générale (I) racémiques a été effectuée en mettant en contact l'agent avec l'-αphénylpropionamide racémique en milieu convenable jusqu'à ce que 20 % du produit soit converti puis en mesurant l'excès énantiomérique.

Les microorganismes qui conviennent particulièrement bien sont choisis parmi Brevibacterium R312 (CBS 717.73) et Corynebacterium N711. (FERM P 4445) ou CorybacteriumN 774 (FERM P 4446) qui permettent d'obtenir des acides aryl-2 alkanoïques S avec un excès énantiomérique en isomère S généralement supérieur à 90 % à partir de l'amide correspondant.

Il est surprenant de noter que, parmi ces microorganismes Brevibacterium R 312, qui est décrit dans FR 7 901 803/2 447 359 et dans Advances in Biochemical Engineering, vol. 14, p. 1-32(1980) comme possédant une amidase générale non stéréospécifique hydrolyse stéréospécifiquement les amides des acides aryl-2 alkanoïques racémiques alors que son mutant A₄ qui possède une amidase L stérospécifique n'hydrolyse pas les amides des acides aryl-2 alkanoïques racémiques.

Il y a lieu de noter que Corynebacterium N 771 et Corybacterium N 774, qui sont décrits dans EP 0 187 680, hydrolysent par exemple le lactronitrile en acide D, L lactique et l'α-aminophénylpropionitrile en D, L-phénylalanine sans observer de stéréosélectivité.

Selon la présente invention, le procédé est généralement mis en oeuvre en milieu aqueux ou hydroorganique homogène ou hétérogène, dans des conditions de température et de pH déterminées en fonction de la nature du microorganisme et de l'enzyme, en agitant une suspension de cellules ou d'extraits cellulaires du microorganisme et de l'amide de l'acide aryloxy-2 ou arylthio-2 alkanoïque racémique.

Le procédé conduit à un mélange de l'acide aryloxy-2 ou arylthio-2 alkanoïque R et de l'amide de l'acide aryloxy-2 ou arylthio-2 alkanoïque S ; enfin de procédé, on sépare l'acide sous forme R de l'aimide sous forme S.

Le microorganisme utilisé possède la propriété d'hydrolyser les nitriles associée à la propriété d'hydrolyser énantiosélectivement l'α -phényl propionamide ; dans ces conditions il est possible d'obtenir un acide phénoxy-2 ou phénylthio-2 alkanoïque R soit à partir du nitrile de l'acide phénoxy-2 ou phénylthio-2 alkanoïque racémique soit à partir de l'amide de l'acide phénoxy-2 ou phénylthio-2 alkanoïque racémique.

Les exemples suivants, donnés à titre non limitatif, montrent comment l'invention peut être mise en pratique.

### EXEMPLE 1

a) La souche Brevibacterium R 312 (CBS 717.73) est cultivée en fiole agitée à 28°C pendant 14 heures dans un milieu dont la composition est la suivante :

| | |
|---|---|
| - Glucose | 10 g |
| - (NH₄)₂SO₄ | 5 g |
| - KH₂ PO₄ | 1,01 g |
| - Nₐ₂HPO₄, 12 H₂O | 1,64 g |
| - K₂HPO₄ | 0,82 g |
| - Ca Cl₂, 2H₂O | 0,012 g |
| - ZnCl₂ | 0,0012 g |
| - FeSO₄, 7H₂O | 0,0012 g |
| - MnSO₄, H₂O | 0,0012 g |
| - MgSO₄, 7H₂O | 0,5 g |
| - Chlorhydrate de thiamine | 0,002 g |
| - eau q.s.p. | 1000 ml |

Cette préculture est utilsée pour ensemencer un milieu de culture ayant la même composition mais contenant du N-méthyl-acétamide a la concentration de 20mM. La culture est effectuée en fiole agitée pendant 24 heures à 28°C. La bionasse obtenue est séparée par centrifugation puis lavée deux fois par une solution de chlorure de sodium a 9 g/litre.
b) Un culot de centrifugation contenant 13 mg de cellules de Brevibacteriuum R 312, exprimés en matières sèches, est mis en suspension dans 2 cm³ de tampon phosphate (50 mM) à pH = 7. On ajoute 25 mg de phényl-2 propionitrile racémique (190 »moles). Apres 24 heures d'agitation à 25°C, le mélange réactionnel est dilue par addition de 23 cm³ d'un mélange acétonitrile-acide chlorhydrique N (90-10 en volumes). Les bacteries sont éliminées par centrifugation. La composition du surnageant est déterminée par chromatographie liquide a haute performance (CLHP).

Le surnageant contient.
119 »mol de phényl-2 propionamide
62 »mol d'acide phényl-2 propionique.

On ajoute du chlorure de sodium pour favoriser la séparation des phases aqueuses ou organiques. Après évaporation à sec de la phase organique, le résidu est repris par 20 cm³ d'un mélange chloroforme-soude 0,1M (1-1 en volumes).

La phase basique est acidifiée puis extraite au chloroforme.

La mesure du pouvoir rotatoire montre que l'excès énantiomérique en isomère S (+) est de 100 %.

Après dérivation de l'acide phényl-2 propionique avec la R (+) α-méthylbenzylamine l'analyse par CLHP montre que l'excès énantiomérique est de 96,4 %.

### EXEMPLE 2

Un culot de centrifugation contenant 1,33 mg de cellules de Brevibacterium R 312 exprimées en matières sèches est mis en suspension dans 2 ml de tampon de phosphate (50 mM) à pH 7,0.

On ajoute 32,6 mg de (hydroxy-4 phénoxy) -2 propionamide racémique (0,180 mmol). Après 24 heures d'agitation à 25°C, le mélange réactionnel est dilué par addition de 23 cm³ d'un mèlange acétonitrile-acide chlorydrique N (90-10 en volumes). Les bactéries sont éliminées par centrifugation. La composition du surnageant est déterminée par chromatographie liquide à haute performance (CLHP).
0,0884 mmol de (hydroxy-4 phénoxy) -2 propionamide
0,0864 mmol d'acide (hydroxy-4 phenoxy) -2 propionique
On ajoute du chlorure de sodium pour favoriser la séparation des phases organiques et aqueuses. Après évaporation à sec de la phase organique, le rèsidu est repris par 10 ml d'acétate d'éthyle plus 6 ml d'une solution d'hydrogénocarbonate de sodium à 10 % p/v dans l'eau. La solution aqueuse, apes decantation est extraite avec deux fois 10 ml d'acetate d'ethyle. La phase aqueuse est acidifiee à pH 1,0 avec une solution d'acide chlorydrique N puis extraite par tois fois par 10 ml d'acétate d'èthyle. Ces phases organiques contenant l'acide sont séchées sur sulfate de sodium et evaporees a sec. Le rèsidu est repris dans l'acétone. La mesure du pouvoir rotatoire de cette solution acétonique montre que l'excès énantiomèrique est de 99 % en isomère R (+).

Après dérivation de l'acide (hydroxy-4 phénoxy) -2 propionique avec la R(+) α méthylbenzylamine, l'analyse CLHP montre que l'excès énantiomérique est de 94 % en isomère R(+).

### EXEMPLE 3

a) On cultive en fiole agitée à 28°C pendant 14 heures la souche Corynebacterium N 771 (FERM P 4445) dans un milieu ayant la composition suivante :

| | |
|---|---|
| - extrait de levure | 3 g |
| - extrait de malt | 3 g |
| - lactopeptone | 5 g |
| - glucose | 10 g |
| - FeSO₄, 7H₂O | 0,1 g |
| - eau q.s.p | 1 litre |

et dont le pH est ajusté à 7,5 par addition de soude avant stérilisation.
Cette préculture est utilisée pour ensemencer au 1/40ème un milieu de même composition.
Après incubation pendant 24 heures à 28°C en fiole agitée, la biomasse est séparée par centrifugation puis lavée deux fois par une solution aqueuse de chlorure de sodium à 9 g/litre.
b) Un culot de centrifugation contenant 11,3 mg de cellules de Corynebacterium N 771, exprimés en matière sèche, est mis en suspension dans 2 cm³ de tampon de phosphate (50 mM) pH 7. On ajoute 34 mg de hydroxy-4 phénoxy)-2 propionamide racémique (0,187 mmol) puis on agite 24 heures à 25°C. Le mélange réactionnel est traité dans les conditions de l'exemple 2.

Le surnageant contient :
0,083 mmol d'(hydroxy-4, phénoxy)-2 propionamide
0,079 mmol d'acide (hydroxy-4, phenoxy)-2 propionique.

Les excès érantiomériques de l'acide (hydroxy-4 phenoxy)-2 propionique mesures par le pouvoir rotatoire et après dérivation avec la R(+) α méthylbenzylamine sont respectivement de 95 % et 91 % en isomère R(+).

### EXEMPLE 4

Les cellules de Corybacterium N774 sont prèparées dans les conditions décrites dans l'exemple 3a/ pour la préparation d'un culot cellulaire de Corynebacterium N771.

Un culot de centrifugation contenant 10,8 mg de cellules de Corynebacterium N774, exprimés en matière sèche, est mis en suspension dans 2 cm³ de tampon phosphate (50 mM) pH 7.

On ajoute 34,5 mg d'(hydroxy-4 phénoxy)-2 propionamide racémique (0,190 mmol) puis on agite 24 heures à 25°C. Le mélange réactionnel est traité dans les conditions de l'exemple 1.

Le surnageant contient :
0,078 mmol d'(hydroxy-4, phènoxy)-2 propioamide
0,080 mmol d'acide (hydroxy-4, phénowy)-2 propionique
Les excès énantiomériques de l'acide (hydroxy-4, phénoxy)-2 propionique mesurés par le pouvoir rotatoire et après dérivation avec la R(+) α methylbenzylamine sont respectivement de 95% et 92% en isomére R(+).

## Revendications

1. Procédé de préparation des énantiomères R (+) des acides aryloxy-2 ou arylthio-2 alkanoïques de formule générale : 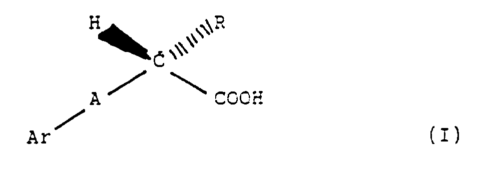 dans laquelle Ar représente un radical aromatique éventuellement substitué, R représente un radical alkyle ayant 1 à 6 atomes de carbone, et A représente un atome d'oxygène ou de soufre caractérisé en ce que :
- on hydrolyse énanthiosélectivement les amides des acides aryloxy-2 ou arylthio-2 alkanoïques racémiques correspondants, éventuellement préparés in situ, en présence d'un microorganisme ou d'une enzyme issue de ce microorganisme, ledit microorganisme étant choisi parmi les Brevibacterium et les Corynebacterium et ayant la propriété de posséder une activité nitrilase associée à la capacité d'hydrolyser énantiosélectivement l'α-phénylpropionamide racémique en acide α-phénylpropionique S avec un excès énantiomérique supérieur à 65 %,
- puis on sépare l'acide aryloxy-2 ou arylthio-2 alkanoïque sous forme R de l'amide de l'acide aryloxy-2 ou arylthio-2 alkanoïque sous forme S.

2. Procédé selon la revendication 1 caractérisé en ce que les microorganismes sont choisis parmi Brevibacterium R 312 (CBS.717.73), Corynebacterium N 771 (FERM P 4445) et Corynebacterium N 774 (FERM P 4446).

3. Procédé selon la revendication 1 caractérisé en ce que les amides de départ sont obtenus par hydrolyse in situ des nitriles des acides aryloxy-2 ou arylthio-2 alkanoïques racémiques en présence du microorganisme utilisé ou de l'enzyme issue de ce microorganisme utilisée.

4. Procédé selon l'une des revendications 1 à 3 pour la préparation des acides de formule générale (II) 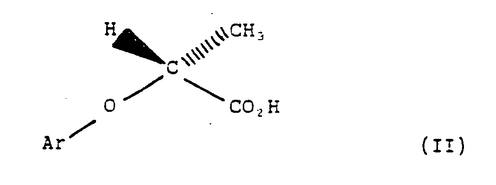 dans laquelle Ar a la même signification que dans la formule (I).

5. Procédé selon la revendication 4 caractérisé en ce que le composé de formule (II) est l'acide (hydroxy-4, phénoxy)-2 propionique sous forme R (+).

## Claims

1. Process for preparing R-(+) enantiomers of 2-(aryloxy)- or 2-(arylthio)alkanoic acids of general formula: 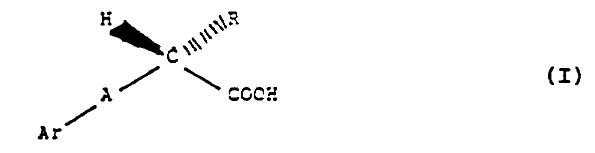 in which Ar denotes an optionally substituted aromatic radical, R denotes an alkyl radical having 1 to 6 carbon atoms and A denotes an oxygen or sulphur atom, characterized in that:
- the amides of the corresponding racemic 2- (aryloxy) - or 2-(arylthio)alkanoic acids, optionally prepared in situ, are enantioselectively hydrolysed in the presence of a microorganism, or an enzyme derived from this microorganism, the said microorganism being chosen from Brevibacterium and Corynebacterium strains and having the property of possessing a nitrilase activity combined with the ability to hydrolyse racemic α-phenylpropionamide enantioselectively to (S) -α-phenylpropionic acid with an enantiomeric excess of more than 65%,
- and the 2- (aryloxy)- or 2- (arylthio)alkanoic acid in the R form is then separated from the amide of the 2- (aryloxy) - or 2- (arylthio)alkanoic acid in the S form.

2. Process according to claim 1, characterized in that the microorganisms are chosen from Brevibacterium R 312 (CBS.717.73), Corynebacterium N 771 (FERM P 4445) and Corynebacterium N 774 (FERM P 4446).

3. Process according to claim 1, characterized in that the starting amides are obtained by in situ hydrolysis of the nitriles of the racemic 2-(aryloxy)- or 2-(arylthio)alkanoic acids in the presence of the microorganism used or of the enzyme derived from this microorganism used.

4. Process according to one of claims 1 to 3 for preparing the acids of general formula (II) 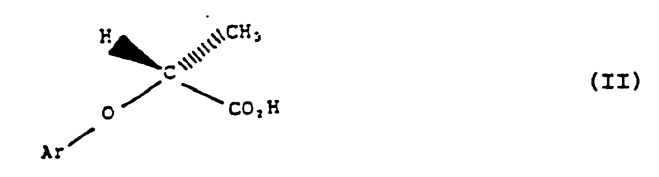 in which Ar has the same meaning as in the formula (I).

5. Process according to claim 4, characterized in that the compound of formula (II) is 2-(4-hydroxyphenoxy)propionic acid in the R-(+) form.

## Patentansprüche

1. Verfahren zur Herstellung der R(+) Enantiomeren der 2-Aryloxy- oder 2 -Arylthioalkansäuren der allgemeinen Formel (I): 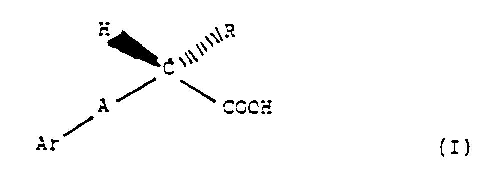 in der Ar eine gegebenenfalls substituierte aromatische Gruppe darstellt, R für eine Alkylgruppe mit 1 bis 6 Kohlenstoffatomen steht, und A für ein Sauerstoff- oder Schwefelatom steht, dadurch gekennzeichnet, daß man:
- enantioselektiv die Amide der entsprechenden racemischen 2-Aryloxy- oder 2-Arylthioalkansäuren, gegebenenfalls in situ hergestellt, in Gegenwart eines Mikroorganismus oder eines aus diesem Mikroorganismus hervorgegangenen Enzyms hydrolysiert, wobei der Mikroorganismus aus Brevibacterium und Corynebacterium ausgewählt wird und eine Nitrilase-Aktivität assoziiert mit der Fähigkeit, enantioselektiv racemisches α-Phenylpropionamid zu S α-Phenylpropionsäure mit einem enantiomeren Überschuß von mehr als 65% zu hydrolisieren besitzt,
- und darauf die 2-Aryloxy- oder 2-Arylthioalkansäure in R-Form von dem Amid der 2-Aryloxy- oder 2 Arylthioalkansäure in S-Form abtrennt.

2. Verfahren nach Anspruch 1, dadurch gekennzeichnet, daß die Mikroorganismen aus Brevibacterium R312 (CBS 717.73), Corynebacterium N771 (FERM P 4445) und Corynebacterium N774 (FERM P 4446) ausgewählt werden.

3. Verfahren nach Anspruch 1, dadurch gekennzeichnet, daß die als Ausgangsverbindungen eingesetzten Amide durch in situ Hydrolyse der Nitrile der racemischen 2-Aryloxy- oder 2-Arylthioalkansäuren in Gegenwart des verwendeten Mikroorganismus oder des aus diesem verwendeten Mikroorganismus hervorgegangenen Enzyms erhalten worden sind.

4. Verfahren nach einem der Ansprüche 1 bis 3 für die Herstellung der Säuren der allgemeinen Formel (II) 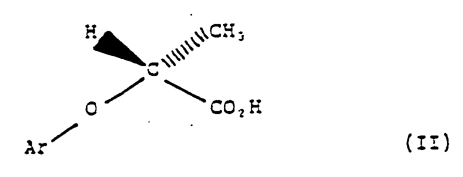 in der Ar die gleiche Bedeutung wie in der Formel (I) hat.

5. Verfahren nach Anspruch 4, dadurch gekennzeichnet, daß die Verbindung der Formel (II) die 2-(4-Hydroxyphenoxy)propionsäure in der R(+)-Form ist.
